(19) Europäisches Patentamt

European Patent Office

Office européen des brevets

(11) **EP 1 341 522 B1**

(12) **EUROPÄISCHE PATENTSCHRIFT**

(45) Veröffentlichungstag und Bekanntmachung des Hinweises auf die Patenterteilung:
**16.11.2005 Patentblatt 2005/46**

(21) Anmeldenummer: **01984822.5**

(22) Anmeldetag: **11.12.2001**

(51) Int Cl.⁷: **A61K 9/16**

(86) Internationale Anmeldenummer:
**PCT/EP2001/014515**

(87) Internationale Veröffentlichungsnummer:
**WO 2002/047664 (20.06.2002 Gazette 2002/25)**

(54) **MIKROPARTIKEL MIT VERBESSERTEM FREISETZUNGSPROFIL UND VERFAHREN ZU DEREN HERSTELLUNG**

MICROPARTICLES WITH AN IMPROVED RELEASE PROFILE AND METHOD FOR THE PRODUCTION THEREOF

MICROPARTICULES PRESENTANT UN MEILLEUR PROFIL DE LIBERATION ET PROCEDE DE FABRICATION

(84) Benannte Vertragsstaaten:
**AT BE CH CY DE DK ES FI FR GB GR IE IT LI LU MC NL PT SE TR**
Benannte Erstreckungsstaaten:
**AL LT LV MK RO SI**

(30) Priorität: **13.12.2000 DE 10061944**
**11.04.2001 DE 10118160**

(43) Veröffentlichungstag der Anmeldung:
**10.09.2003 Patentblatt 2003/37**

(73) Patentinhaber: **MERCKLE GMBH**
**89143 Blaubeuren (DE)**

(72) Erfinder:
• **KISSEL, Thomas**
**79219 Staufen im Breisgau (DE)**
• **FRIDRICH, Ruland**
**89143 Blaubeuren (DE)**
• **SCHNEIDER, Peter**
**65599 Dornburg-Thalheim (DE)**

(74) Vertreter: **Teipel, Stephan et al**
**Lederer & Keller**
**Patentanwälte**
**Prinzregentenstrasse 16**
**80538 München (DE)**

(56) Entgegenhaltungen:
**EP-A- 0 669 128          EP-A- 1 044 683**
**WO-A-00/40221          WO-A-94/23699**
**WO-A-99/36071          CA-A- 2 213 906**
**FR-A- 2 702 968          US-A- 5 401 502**
**US-A- 5 700 486          US-A- 5 849 884**
**US-A- 5 942 253          US-A- 5 980 947**

• **MCGEE J.P.; DAVIS S.S.; O'HAGAN D.T.: 'Zero order release of protein from poly (d,l-lactide-co-glycolide) microparticles prepared using a modified phase separation technique' JOURNAL OF CONTROLLED RELEASE Bd. 34, Nr. 2, 01 Mai 1995, AMSTERDAM, Seiten 77 - 86, XP004037506**

**Beschreibung**

Die vorliegende Erfindung betrifft Mikropartikel zur verzögerten Freisetzung eines physiologisch aktiven Wirkstoffs, die wenigstens einen Wirkstoff und eine polymere Matrix enthalten. Die erfindungsgemäßen Mikropartikel besitzen eine besonders vorteilhafte Freisetzungscharakteristik. Die Erfindung betrifft auch Verfahren zur Herstellung derartiger Mikropartikel.

Bei der Verabreichung von Arzneimitteln ist es oftmals wünschenswert, über einen längeren Zeitraum einen möglichst konstanten Plasmaspiegel des Wirkstoffs zu erhalten. Dies ist insbesondere dann schwer zu erreichen, wenn der entsprechende Wirkstoff im Körper schnell abgebaut oder ausgeschieden wird. Um wiederholte Applikationen in kurzen Zeitabständen zu vermeiden, wurden verschiedene Depotarzneiformen vorgeschlagen, die zum Ziel haben, über einen längeren Zeitraum eine möglichst konstante Menge an Wirkstoff freizusetzen. Derartige Depotarzneiformen haben oftmals die Form von Mikropartikeln, die parenteral verabreicht werden können, beispielsweise als Implantat oder durch subkutane Injektion. In der Regel umfassen derartige Arzneiformen eine Polymermatrix, in der der Wirkstoff verteilt ist ("microspheres"), oder einen wirkstoffhaltigen Kern, der von einer polymerhaltigen Schicht umgeben ist (Mikrokapseln).

Im Stand der Technik sind verschiedene Verfahren bekannt, um Mikropartikel herzustellen.

Beim sogenannten W/O/W-Verfahren wird zunächst eine den Wirkstoff enthaltende wäßrige Phase (W1) in einer organischen Lösung des Polymers (O) dispergiert, die entstehende W1/O-Emulsion wird dann in einer weiteren wäßrigen Phase (die sogenannte äußere Phase; W2) dispergiert. Das Polymer wird durch das Entfernen des organischen Lösungsmittels koazerviert und bildet Mikropartikel. Durch das jeweilige Dispergierverfahren kann die Partikelgröße beeinflußt werden. Die Entstehung der Mikropartikel hängt schließlich noch von der Möglichkeit des Abdampfens des Lösungsmittels ab. Deshalb wird das W/O/W-Doppelemulsionsverfahren auch als "solvent evaporation/ extraction method/technique" bezeichnet. Nach Aushärten der Mikropartikel und Entfernen der Lösungsmittel erhält man Mikropartikel, die den Wirkstoff enthalten. Oft enthalten derartige Mikropartikel viskositätserhöhende Substanzen wie z.B. Gelatine.

Im Stand der Technik sind ebenfalls S/O/W-Verfahren bekannt, bei denen der Wirkstoff nicht in einer wäßrigen Lösung vorliegt, sondern als Feststoff (S). Der Feststoff wird dann direkt in der organischen Phase (O) dispergiert. Die weiteren Schritte entsprechen dem W/O/W-Verfahren.

Schließlich gibt es sogenannte S/O/O-Verfahren, bei denen die äußere Phase keine wäßrige Phase ist, sondern eine nicht-wäßrige Phase, die ein Schutzkolloid oder einen Emulgator enthält.

Es ist wünschenswert, die an den Patienten zu verabreichende Menge an Mikropartikeln möglichst gering zu halten. Beispielsweise sollte das zu injizierende Volumen an Mikropartikeln möglichst gering sein, damit unter anderem die Schmerzen bei der Injektion geringer sind. Daher sollte der Wirkstoffgehalt in den Mikropartikeln möglichst hoch sein. Die Wirkstoffbeladung ist ein wichtiges Charakteristikum von Mikropartikeln. Man unterscheidet zwischen praktischem und theoretischem Beladungsgrad. Als Synonyme für den praktischen Beladungsgrad werden auch die Ausdrücke effektiver Beladungsgrad oder effektiver Wirkstoffgehalt verwendet. Der theoretische Beladungsgrad ist wie folgt definiert:

$$\text{Theoret. Beladungsgrad in \%} = \frac{\text{Masse Wirkstoff} \times 100}{\text{Masse (Wirkstoff + Polymer + Zuschlagsstoffe)}}$$

Dabei handelt es sich um die Masse der während der Herstellung eingesetzten Bestandteile. Der effektive wirkstoffgehalt ist wie folgt definiert:

$$\text{Effektiver Wirkstoffgehalt in \%} = \frac{\text{Masse Wirkstoff in mg} \times 100}{\text{Einwaage an Mikropartikeln in mg}}$$

Das Verhältnis aus effektivem Wirkstoffgehalt und theoretischem Beladungsgrad wird als Verkapselungsausbeute bezeichnet. Die Verkapselungsausbeute ist ein wichtiger Prozeßparameter und ein Maß für die Effektivität des Verfahrens:

$$\text{Verkapselungsausbeute in \%} = \frac{\text{effektiver Wirkstoffgehalt} \times 100}{\text{theoretischer Beladungsgrad}}$$

Ebenfalls ein bedeutendes Kriterium ist das Freisetzungsprofil der Mikropartikel. Die Wirkstofffreisetzung kann zeitlich grob in drei Phasen unterteilt werden. In einer anfänglichen "Burst"-Phase werden üblicherweise in relativ kurzer Zeit erhebliche Mengen des in den Mikropartikeln enthaltenen Wirkstoffs freigesetzt. Zum Teil handelt es sich hierbei um Wirkstoff, der sich auf der oder nahe der Oberfläche der Partikel befindet. Die Menge des in der "Burst"-Phase freigesetzten Wirkstoffs sollte möglichst gering sein. In der sich anschließenden "Lag"-Phase ist bei den bisher im Stand der Technik bekannten Zubereitungen die Wirkstofffreisetzung vernachlässigbar gering, insbesondere beim Einsatz von PLGA-Polymeren als Matrixbildner. Es wäre wünschenswert, daß während der "Lag"-Phase eine über den Freisetzungszeitraum möglichst konstante Wirkstoffabgabe erfolgt. In der abschließenden Phase der Bioerosion werden die Partikel hydrolysiert und geben so durch starken Massenverlust und Molekulargewichtsverlust verstärkt Wirkstoff frei. Idealerweise würde bereits während der "Lag"-Phase die gesamte Wirkstoffmenge freigesetzt werden.

Kishida et al. (1990) J. Controlled Release 13, 83-89 untersuchen den Einfluß von Beladungsgrad, Wirkstofflipophilie und Lösungsmittelentfernungsrate an der lipophilen Substanz Sudan III verglichen mit dem polaren Etoposid. Es zeigte sich beim Einsatz von Polyvinylalkohol als Stabilisator, daß die Entfernung des Lösungsmittels während der Aushärtungsphase mittels verschiedener Vakuumeinstellungen keinen Einfluß auf die Freisetzung hat.

In Cleland et al. (1997) J. Controlled Release 47, 135-150 wird für ein W/O/W-Verfahren mit PLGA zur Verkapselung von gp120 der Einfluß der kinematischen Viskosität des Polymers in der Primäremulsion und der Gebrauch von überschüssigem Dichlormethan in der äußeren Phase auf Wirkstoffbeladung und -freisetzung während der "Burst"-Phase untersucht.

WO 00/40221 offenbart Mikropartikel aus einer Polymermatrix, welche ein sigmoidales Freisetzungsprofil aufweisen.

Es ist eine Aufgabe der vorliegenden Erfindung, Mikropartikel bereitzustellen, die ein vorteilhaftes Freisetzungsprofil aufweisen.

Überraschenderweise wurde gefunden, daß Mikropartikel mit einer höheren Gesamtfreisetzung erhalten werden, wenn die äußere Phase, zu der die Primäremulsion gegeben wird, vorgekühlt wird. In der vorliegenden Anmeldung gilt als verwertbare Gesamtfreisetzung der prozentuale Anteil der in den Mikropartikeln enthaltenen Gesamtmenge an Wirkstoff, der innerhalb von 900 Stunden ab dem Beginn der Freisetzung freigesetzt wird. Ebenfalls wurde gefunden, daß die Menge des während der "Burst"-Phase freigesetzten Wirkstoffs signifikant reduziert werden kann, indem das organische Lösungsmittel beschleunigt entfernt wird. Dies geschieht entweder dadurch, daß nach Dispergierung der Primäremulsion in der äußeren Phase die entstehende Emulsion oder Dispersion einem niedrigen Druck ausgesetzt wird oder dadurch, daß ein inertes Gas durch die entstehende Emulsion oder Dispersion geleitet wird, was zu einer schnelleren Entfernung des organischen Lösungsmittels führt.

Die vorliegende Erfindung betrifft also ein Verfahren zur Herstellung von Mikropartikeln zur verzögerten Freisetzung eines Wirkstoffs, dadurch gekennzeichnet, daß

a) eine den Wirkstoff enthaltende Zusammensetzung zu einer organischen Lösung eines Polymers gegeben wird und darin dispergiert wird,

b) die in a) entstehende Emulsion oder Dispersion in eine äußere Phase gegeben wird und darin dispergiert wird, wobei die äußere Phase zum Zeitpunkt der Zugabe eine Temperatur von 0°C bis 20°C hat, und

c) das organische Lösungsmittel entfernt wird, indem die in b) entstehende Dispersion oder Emulsion einem Druck von weniger als 1.000 mbar ausgesetzt wird, oder indem ein inertes Gas in die in b) entstehende Dispersion oder Emulsion eingeleitet wird.

Als Wirkstoffe in den Mikropartikeln können alle physiologisch aktiven Wirkstoffe eingesetzt werden. Bevorzugt sollte es sich um wasserlösliche Substanzen handeln. Beispiele für Wirkstoffe, die verwendet werden können, sind Impfstoffe, Antitumormittel, Antipyretika, Analgetika, antiinflammatorische Substanzen, Wirkstoffe, die einen Einfluß auf die Blutgerinnung haben, wie beispielsweise Heparin, Antitussiva, Sedativa, Muskelrelaxantien, Antiulcerativa, Antiallergika, Vasodilatatoren, antidiabetische Substanzen, Antituberkulosemittel, Hormonpräparate, Kontrazeptiva, Hemmstoffe der Knochenresorption, Angiogeneseinhibitoren, usw. Üblicherweise werden als Wirkstoffe Peptide oder Proteine verwendet. Beispiele für mögliche Peptid- oder Proteinwirkstoffe sind Salmon-Calcitonin (sCT), Lysozym, Cytochrom C, Erythropoietin (EPO), luteinizing hormone releasing hormone (LHRH), Buserelin, Goserelin, Triptorelin, Leuprorelin, Vasopressin, Gonadorelin, Felypressin, Carbetocin, Rinderserumalbumin (BSA), Oxytocin, Tetanustoxoid, Bromocriptin, growth hormone releasing hormone (GHRH), Somatostatin, Insulin, tumor necrosis factor (TNF), colony stimulating factor (CSF), epidermal growth factor (EGF), nerve growth factor (NGF), Bradykinin, Urokinase, Asparaginase, Neurotensin, Substanz P, Kallikrein, gastric inhibitory polypeptide (GIP), growth hormone releasing factor (GRF), Prolactin, adrenocorticotropes Hormon (ACTH), thyrotropin releasing hormone (TRH), thyroid stimulating hormone (TSH), melanocyte stimulating hormone (MSH), Parathormon (LH), Gastrin, Glucagon, Enkephalin, bone morphogenetic protein (BMP), $\alpha$-, $\beta$-, $\gamma$-Interferon, Angiotensin, Thymopoetin und thymic humoral factor (THF).

Wirkstoffe, die Peptide bzw. Proteine sind, können aus einer natürlichen Quelle stammen oder aber rekombinant hergestellt und isoliert werden. Rekombinant hergestellte Wirkstoffe können sich von den entsprechenden nativen Wirkstoffen unterscheiden, beispielsweise in der Art und dem Umfang der posttranslationalen Modifikationen, aber auch in der Primärsequenz. Derart veränderte Wirkstoffe können andere Eigenschaften besitzen, wie veränderte pharmakologische Wirksamkeit, verändertes Ausscheidungsverhalten, usw. Alle derartigen "Varianten" natürlicher Wirkstoffe sind von der Erfindung umfaßt. Weitere mögliche Wirkstoffe sind Heparin und Nukleinsäuren wie DNA- und RNA-Moleküle. Die DNA-Moleküle können in linearer oder zirkulärer Form vorliegen. Es kann sich auch um Plasmide oder Vektoren, insbesondere Expressionsvektoren handeln. Ein Beispiel ist der in WO 98/51321 beschriebene Expressionsvektor pcDNA3.

Schließlich sind auch virale Vektoren umfaßt, die zur Gentherapie eingesetzt werden. Dabei können auch Komplexe aus Chitosan, Natrium-Alginat oder anderen kationischen Polymeren, wie Polyethylenimin oder Poly(Lysin) oder anderen kationischen Aminosäuren eingesetzt werden. Die eingesetzte Nukleinsäure kann einzel- oder doppelsträngig sein. Einzelsträngige DNA kann beispielsweise in Form von Antisense-Oligonukleotiden verwendet werden. Es können auch "nackte" Nukleinsäurefragmente eingesetzt werden; in diesen Fällen ist die Nukleinsäure nicht mit anderen Stoffen verbunden.

Die Wirkstoffkonzentration ist unter anderem abhängig vom jeweiligen Wirkstoff und der Art der Behandlung, für die sie verwendet werden sollen. Peptid-/Proteinwirkstoffe werden in der Regel in einer Konzentration von 0,01 bis 30% eingesetzt, bevorzugt von 0,5 bis 15%, vor allem 1,0 bis 7,5%, bezogen auf die eingesetzte Polymermasse.

Die organische, nicht mit Wasser mischbare Phase dient zum Lösen des biologisch abbaubaren Polymers. Dabei wird das Polymer in einem geeigneten organischen Lösungsmittel gelöst, in dem der Wirkstoff nicht löslich ist. Beispiele für derartige organische Lösungsmittel sind Ethylacetat, Aceton, Dimethylsulfoxid, Toluol, Chloroform, Ethanol, Methanol, usw. Besonders bevorzugt ist Dichlormethan. Die Konzentration des Polymers in der organischen Phase ist üblicherweise höher als 5% (w/v), bevorzugt 5 bis 50%, am bevorzugtesten 15 bis 40%.

Als Polymere, die die Polymermatrix der Mikropartikel bilden, können alle bioabbaubaren und biologisch verträglichen polymere verwendet werden. Diese können natürlichen oder synthetischen Ursprungs sein. Beispiele für Polymere natürlichen Ursprungs sind Albumin, Gelatine, Carragen. Beispiele für synthetische Polymere, die in dem erfindungsgemäßen Verfahren verwendet werden können, sind Polymere aus Fettsäuren (z.B. Polymilchsäure, Polyglykolsäure, Polyzitronensäure, Folyäpfelsäure, Polymilchsäurecaprolacton, usw.), Poly-α-cyanoacrylsäureester, Poly-β-hydroxybuttersäure, Polyalkylenoxalate (z.B. Polytrimethylenoxalat, Polytetramethylenoxalat, usw.), Polyorthoester, Polyorthocarbonate und andere Polycarbonate (z.B. Polyethylencarbonat, Polyethylenpropylencarbonat, usw.), Polyaminosäuren (z.B. Poly-γ-benzyl-L-glutaminsäure, Poly-L-alanin, Poly-γ-methyl-L-glutaminsäure, usw.), und Hyaluronsäureester, usw. Weitere bioverträgliche Copolymere sind Polystyrol, Polymethacrylsäure, Copolymere aus Acrylsäure und Methacrylsäure, Polyaminosäuren, Dextranstearat, Ethylcellulose, Acetylcellulose, Nitrocellulose, Maleinsäureanhydrid-Copolymere, Ethylen-VinylacetatCopolymere, wie Polyvinylacetat, Polyacrylamid, usw. Die genannten Polymere können allein oder in Kombination miteinander verwendet werden. Sie können in Form von Copolymeren oder als eine Mischung von zwei oder mehreren der Polymere verwendet werden. Auch ihre Salze können eingesetzt werden. Unter den genannten Polymeren sind Milchsäure/Glykolsäure-Copolymere (PLGA) bevorzugt. Bevorzugt sind PLGA-Polymere mit einer Zusammensetzung von 0:100 bis 100:0 Milchsäure zu Glykolsäure und einem Molekulargewicht von 2.000 bis 2.000.000 Da. Besonders bevorzugt sind PLGA-Polymere mit einem Molekulargewicht von 2.000 bis 200.000 Da und einem Verhältnis Milchsäure zu Glykolsäure von 25:75 bis 75:25 oder 50:50. Es können dabei auch L-PLA oder D,L-PLA oder Mischungen dieser oder Copolymere davon eingesetzt werden.

Die den Wirkstoff enthaltende Zusammensetzung kann eine wäßrige Lösung sein, beispielsweise bei Anwendung des W/O/W-Verfahrens. Dann wird üblicherweise der Wirkstoff in Wasser oder einer Pufferlösung gelöst und direkt in die organische Polymerlösung dispergiert. Die entstehende WI/O- oder Primäremulsion wird dann in die gegebenenfalls ein Schutzkolloid enthaltende äußere wäßrige Phase (W2) eingespritzt und mit üblichen Hilfsmitteln dispergiert. Nach diesem Schritt entsteht die Doppelemulsion oder W1/O/W2-Emulsion. Nach einer Aushärtungsphase werden die entstandenen Mikropartikel von der äußeren wäßrigen Phase separiert und können danach lyophilisiert werden. Bei großem W1-Volumen und niedriger Viskosität der Polymerlösung werden beim W/O/W-Verfahren Mikrokapseln erhalten. Beispielsweise würde ein Volumenverhältnis W1:O:W2 von 1:10:1000 zur Bildung von "microspheres", ein Volumenverhältnis von 9:10:1000 zur Bildung von Mikrokapseln führen.

Die den Wirkstoff enthaltende Zusammensetzung kann aber auch in Feststofform vorliegen. Dabei wird der Wirkstoff in fester Form direkt in die organische Polymerlösung dispergiert. Die weiteren Herstellungsschritte entsprechen denen des W/O/W-Verfahrens. Durch weitere Verfahrensschritte kann entweder ein S/O/W- oder S/O/O-Verfahren zur Anwendung kommen.

In bestimmten Ausführungsformen des erfindungsgemäßen Verfahrens ist die äußere Phase eine wäßrige Lösung (W2). Diese wäßrige Lösung kann einen Emulgator oder ein Schutzkolloid enthalten. Beispiele für Schutzkolloide sind Polyvinylalkohol, Polyvinylpyrrolidon, Polyethylenglykol, usw. Bevorzugt ist Polyvinylalkohol. Beispielsweise können verschiedene von der Firma Clariant erhältliche Polyvinylalkohole eingesetzt werden wie Mowiol® 18-88, Mowiol® 4-88, Mowiol® 47-88 oder Mowiol® 20-98. Die Schutzkolloide werden üblicherweise in einer Konzentration von 0,01% bis 10% eingesetzt, bevorzugt sind 0,01% bis 5%. Das Molekulargewicht der Schutzkolloide kann zwischen 2.000 und 1.000.000 Da, bevorzugt zwischen 2.000 und 200.000 Da, liegen. Die Volumina von W1/O-Primäremulsion und äußerer Phase sollten ein Verhältnis von 1:5 bis 1:1.000 zueinander aufweisen.

Alternativ kann als äußere Phase auch eine sogenannte "ölige" Phase zum Einsatz kommen, die nicht mit der Primäremulsion mischbar ist ("W/O/O- bzw. S/O/O-Verfahren"). Beispielsweise können Silikonöl oder Paraffinöl eingesetzt werden, die einen Emulgator und/oder ein Schutzkolloid enthalten. Im Gegensatz zum Einsatz einer wäßrigen äußeren Phase muß bei Verwendung einer "öligen" äußeren Phase ein Emulgator oder Schutzkolloid enthalten sein. Beispiele für Emulgatoren in der äußeren öligen Phase sind Span, Tween oder Brij, vorzugsweise in einer Konzentration von 0,01 bis 10 Gew.-%.

Erfindungsgemäß hat die äußere Phase eine Temperatur von 0 bis 20°C, wenn die Primäremulsion zur äußeren Phase gegeben wird und darin dispergiert wird. Vorzugsweise beträgt diese Temperatur 0°C bis 10°C, bevorzugter 3°C bis 7°C, am bevorzugtesten ungefähr 5°C. Es ist ebenfalls bevorzugt, daß die dabei entstehende Emulsion oder Dispersion anschließend weiterhin in den genannten Temperaturbereichen temperiert wird, beispielsweise in einem Laborreaktor. Am bevorzugtesten wird nach Dispersion der Primäremulsion in der äußeren Phase bis zum Ende der Aushärtung der Mikropartikel die erfindungsgemäße Temperatur eingehalten.

Nach dem erfindungsgemäßen Verfahren wird auch das organische Lösungsmittel beschleunigt entfernt. Das kann dadurch bewerkstelligt werden, daß die Emulsion oder Dispersion, die durch Dispersion der Primäremulsion in der äußeren Phase entsteht, einem Unterdruck ausgesetzt wird, das heißt einem Druck, der geringer ist als Atmosphärendruck. Erfindungsgemäß kann die Emulsion oder Dispersion einem Druck von weniger als 1.000 mbar ausgesetzt werden, vorzugsweise einem Druck von 500 mbar oder weniger, am bevorzugtesten einem Druck von 50 bis 150 mbar. Durch dieses Vakuum wird das organische Lösungsmittel schneller entfernt. Das Vakuum läßt sich vorteilhaft während der Aushärtung der Mikropartikel anlegen, wenn ein Laborreaktor zur Herstellung der Mikropartikel eingesetzt wird. Als Alternative zum Anlegen eines Unterdrucks kann das organische Lösungsmittel auch beschleunigt entfernt werden, indem ein inertes Gas in die Emulsion oder Dispersion eingeleitet wird. Als inerte Gase können beispielsweise Edelgase verwendet werden, bevorzugt ist jedoch Stickstoff. Durch das Einblasen von Stickstoff wird das flüchtige organische Lösungsmittel schneller entfernt.

In einer besonders bevorzugten Ausführungsform erfolgt die Aushärtung der Mikropartikel bei einer niedrigen Temperatur, das heißt in einem Temperaturbereich zwischen etwa 0°C und etwa 10°C, bevorzugt um etwa 5°C und unter reduziertem Druck, das heißt bei einem Druck von 500 mbar oder weniger. Besonders bevorzugt wird hierbei ein Vakuum angelegt, das heißt ein Druck zwischen etwa 50 und etwa 100 mbar.

Es wurde auch gefunden, daß die Anwesenheit von Chitosan in Mikropartikeln höhere Beladungsgrade an Wirkstoff ermöglicht als bei Mikropartikeln des Standes der Technik. Zur Herstellung der Mikropartikel der vorliegenden Erfindung kann somit auch Chitosan verwendet werden. Chitosan ist ein Polymer, das durch Deacetylierung von Chitin, einem in Insekten und Krebsen vorkommenden Polysaccharid, erhältlich ist. Es ist üblicherweise ein Polysaccharid mit einer linearen Kette, die aus 2-Amino-2-desoxy-β-D-glucopyranose (GlcN) aufgebaut ist, wobei die Monomere β-(1,4)-verknüpft sind (100% Deacetylierung). Bei einer unvollständigen Deacetylierung entstehen aber Chitosanpräparationen, die noch unterschiedliche Anteile an 2-Acetamido-2-desoxy-β-D-glucopyranose (GlcNAc) in der Polysaccharidkette aufweisen.

Erfindungsgemäß kann das Chitosan verschiedene Deacetylierungsgrade aufweisen. Ein zu praktisch 100% deacetyliertes Chitosan enthält im wesentlichen nur noch GlcN und kein GlcNAc mehr. Vorzugsweise hat das erfindungsgemäße Chitosan einen Deacetylierungsgrad von 25 bis 100%, am bevorzugtesten von 50 bis 100%.

Das Gewichtsverhältnis von physiologisch aktivem Wirkstoff zu Chitosan ist vorzugsweise 1:0,01 bis 1:25, bevorzugter 1:0,01 bis 1:10, am bevorzugtesten 1:1. Das Verhältnis ist angegeben in Gew./Gew.

Üblicherweise wird Chitosan mit einem Molekulargewicht von 10.000 bis 2.000.000 Da verwendet, vorzugsweise mit 40.000 bis 400.000 Da. Meist wird das Chitosan in einer 0,001% bis 70%igen Essigsäure gelöst, bevorzugt in einer 0,01% bis 10%igen Essigsäure (m/m). Erfindungsgemäß können die Partikel durch W/O/W-, S/O/W- oder S/O/O-Verfahren hergestellt werden. Der Wirkstoff kann mit Chitosan in Essigsäure gelöst werden oder zunächst in Wasser gelöst und dann mit dem gelösten Chitosan dispergiert werden. Dieses Chitosan-Wirkstoffgel wird dann direkt in die organische Polymerlösung dispergiert (W/O/W). Die Chitosan-Wirkstoff-Lösung kann auch sprühgetrocknet werden und das feste Pulver dann direkt in die organische Polymerlösung dispergiert werden (S/O/W; S/O/O).

Die Konzentration von Chitosan in der inneren Phase beim W/O/W-Verfahren ist im allgemeinen 0,01% bis 50% Chitosan, bezogen auf die Polymermasse, bevorzugt aber 0,01% bis 25% Chitosan, bezogen auf die Polymermasse. Das Gewichtsverhältnis von physiologisch aktivem Wirkstoff zu Chitosan sollte 1:0,01 bis 1:25, bevorzugt 1:0,1 bis 1:10, am bevorzugtesten 1:1, sein. Wird das S/O/W-Verfahren angewendet, sollte eine Konzentration von Chitosanwirkstoffkomplex von 0,01% bis 50%, bevorzugt 0,1% bis 25%, bezogen auf die Polymermasse, eingesetzt werden.

Die Erfindung betrifft auch Mikropartikel, die durch das erfindungsgemäße Verfahren hergestellt werden können. Derartige Mikropartikel weisen vorteilhafte Eigenschaften in ihrem Freisetzungsprofil auf. So ist die Menge an Wirkstoff, die während der "Burst"-Phase freigesetzt wird, sehr niedrig. Ebenfalls wird ein Großteil des in den Mikropartikeln enthaltenen Wirkstoffs während der "Lag"-Phase freigesetzt. Die Gesamtfreisetzung an Wirkstoff ist also sehr hoch. Die vorliegende Erfindung betrifft somit Mikropartikel enthaltend eine Polymermatrix und wenigstens einen physiologisch aktiven Wirkstoff, dadurch gekennzeichnet, daß gemäß dem in vitro-Freisetzungsprofil der Mikropartikel

a) innerhalb von 24 Stunden ab dem Beginn der Freisetzung weniger als 25% der gesamten Wirkstoffmenge freigesetzt werden; und

b) innerhalb von 900 Stunden ab dem Beginn der Freisetzung wenigstens 80% der gesamten Wirkstoffmenge freigesetzt worden sind.

Angaben über die Freisetzung von Wirkstoff in dieser Anmeldung beziehen sich auf die in vitro ermittelte Freisetzung in einem Freisetzungsgerät gemäß dem in Beispiel 5 dargestellten Verfahren. Es ist bekannt, daß die Freisetzung an Wirkstoff in diesem in vitro-Verfahren der Freisetzung in vivo nahe kommt.

Mikropartikel mit einem derart vorteilhaften Freisetzungsprofil sind bisher im Stand der Technik nicht bekannt. Die Mikropartikel aus dem Stand der Technik weisen eine höhere Freisetzung während der "Burst"-Phase und/oder eine sehr geringe Freisetzung während der "Lag"-Phase auf, so daß die Gesamtfreisetzung niedrig ist. Dadurch besteht die Gefahr, daß erst während der sich anschließenden Bioerrosionsphase wiederum eine hohe Menge an Wirkstoff freigesetzt wird.

Die erfindungsgemäßen Mikropartikel setzen innerhalb von 24 Stunden ab dem Beginn der Freisetzung weniger als 25% der gesamten Wirkstoffmenge frei, vorzugsweise weniger als 20%, am bevorzugtesten weniger als 15%.

Ebenfalls haben die Mikropartikel die Eigenschaft, daß innerhalb von 900 Stunden ab dem Beginn der Freisetzung wenigstens 80% der gesamten enthaltenen Wirkstoffmenge freigesetzt werden, vorzugsweise wenigstens 85%, am bevorzugtesten wenigstens 90%.

Die erfindungsgemäßen Mikropartikel zeigen eine Freisetzung, die in dem Zeitraum zwischen 48 Stunden und 900 Stunden nach Beginn der Freisetzung, vorzugsweise in dem Zeitraum zwischen 24 Stunden und 900 Stunden nach Beginn der Freisetzung, im wesentlichen nach einer Kinetik nullter Ordnung erfolgt. Das bedeutet, daß über einen Zeitraum von mehr als 30 Tagen täglich eine im wesentlichen konstante Wirkstoffmenge freigesetzt wird. Vorzugsweise wird in dem Zeitraum zwischen 48 Stunden und 900 Stunden nach Beginn der Freisetzung täglich 1,5% bis 2,5% der gesamten Wirkstoffmenge freigesetzt, vorzugsweise 2% bis 2,5%.

Die erfindungsgemäßen Mikropartikel haben üblicherweise einen Durchmesser von 1 bis 500 μm, vorzugsweise von 1 bis 200 μm, noch bevorzugter von 1 bis weniger als 150 μm, am bevorzugtesten von 1 bis 100 μm. Sie können im wesentlichen kugelförmig sein oder eine andere Form aufweisen. Falls die Partikel nicht kugelförmig sind, ist unter dem Durchmesser die größte räumliche Ausdehnung eines Partikels zu verstehen. Die Polymermatrix kann dabei als Hülle ausgebildet sein, die einen Kern umgibt, oder als ein den gesamten Partikel durchziehendes "Gerüst". Die Mikropartikel der vorliegenden Erfindung umfassen daher sowohl Partikel, die einen wirkstoffhaltigen Kern, umgeben von einer polymeren Schicht, aufweisen (Mikrokapseln), als auch Partikel, die eine Polymermatrix aufweisen, in der der Wirkstoff verteilt ist ("microspheres").

In einer besonderen Ausführungsform können die Mikropartikel auch Chitosan enthalten. Die Eigenschaften und erfindungsgemäßen Konzentrationen von Chitosan sind wie oben angegeben. Derartige Partikel zeigen einen höheren effektiven Beladungsgrad an Wirkstoff.

Ein weiterer Aspekt der vorliegenden Erfindung ist ein Arzneimittel, das die erfindungsgemäßen Mikropartikel, gegebenenfalls mit pharmazeutisch verträglichen Hilfsstoffen, umfaßt.

Die vorliegende Erfindung stellt zum ersten Mal Mikropartikel zur Verfügung, die eine niedrige Wirkstofffreisetzung während der "Burst"-Phase mit einer hohen Gesamtfreisetzung kombinieren. Darüber hinaus zeigen die erfindungsgemäßen Mikropartikel einen im wesentlichen linearen Verlauf der Wirkstofffreisetzung während der "Lag"-Phase. Durch die erfindungsgemäßen Mikropartikel ist eine Wirkstofffreisetzung über Wochen und sogar Monate möglich. Sie eignen sich daher insbesondere für eine subkutane/intramuskuläre Applikation.

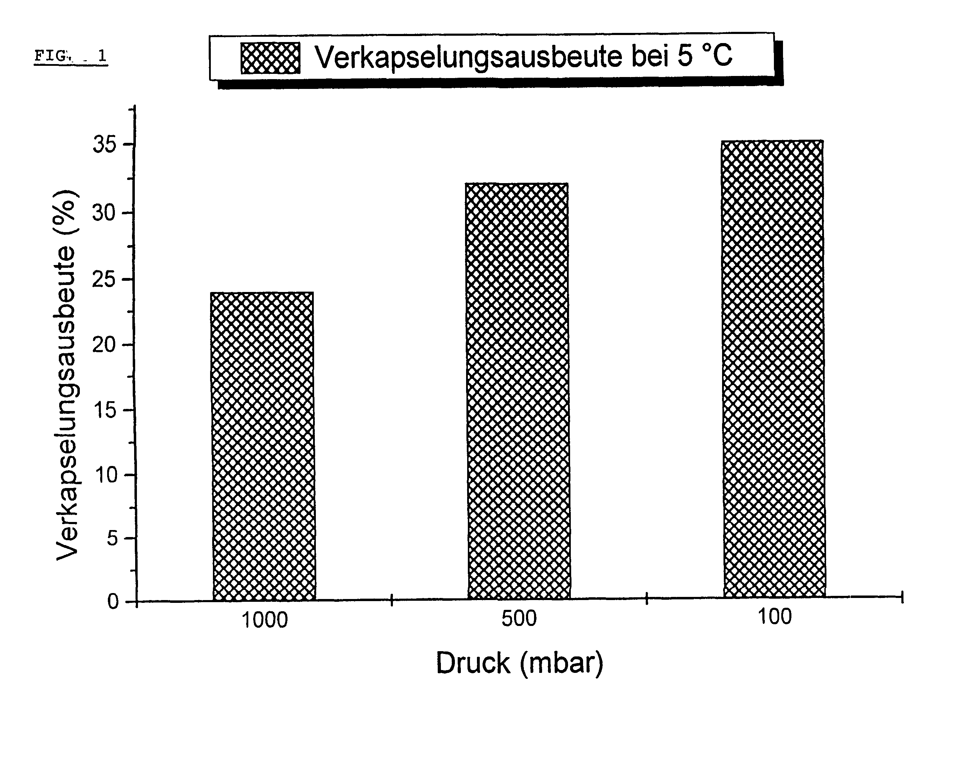

Figur 1 zeigt die Abhängigkeit der Verkapselungsausbeute (VE) vom eingesetzten Druck während der Aushärtung der Mikropartikel im Laborreaktor bei konstant 5°C. Die Verkapselungsausbeute steigt mit sinkendem Druck.

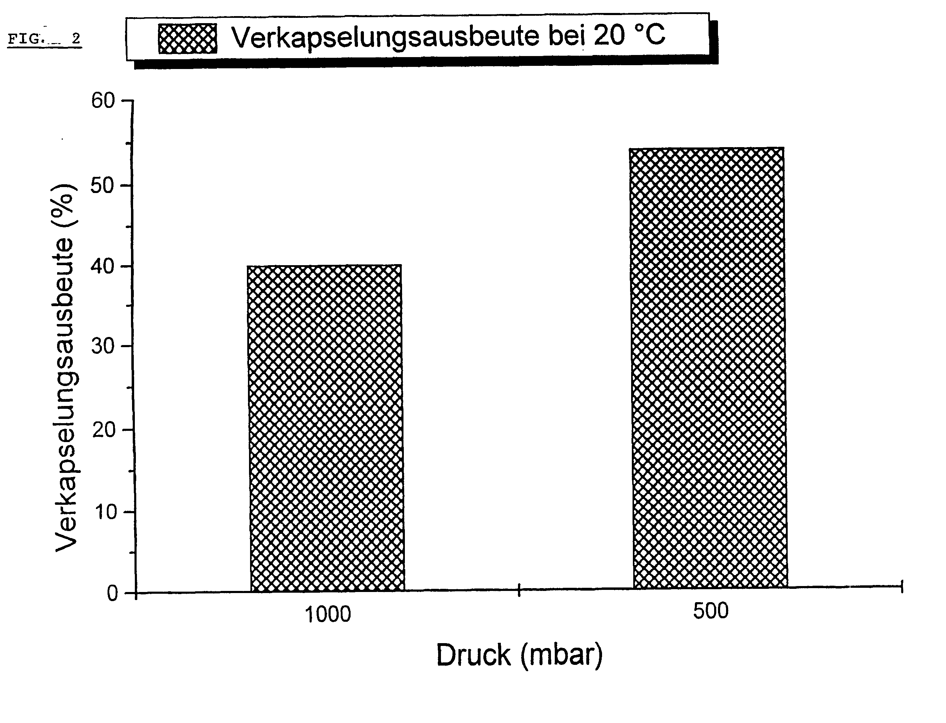

Figur 2 zeigt die Abhängigkeit der Verkapselungsausbeute (VE) vom eingesetzten Druck während der Aushärtung der Mikropartikel im Laborreaktor bei konstant 20°C. Im Gegensatz zu Figur 1 werden hier nur zwei Drücke, nämlich Atmosphärendruck und 500 mbar, untersucht. Auch bei 20°C ist erkennbar, daß ein niedrigerer Druck während der Aushärtung zu einer höheren Verkapselungsausbeute führt.

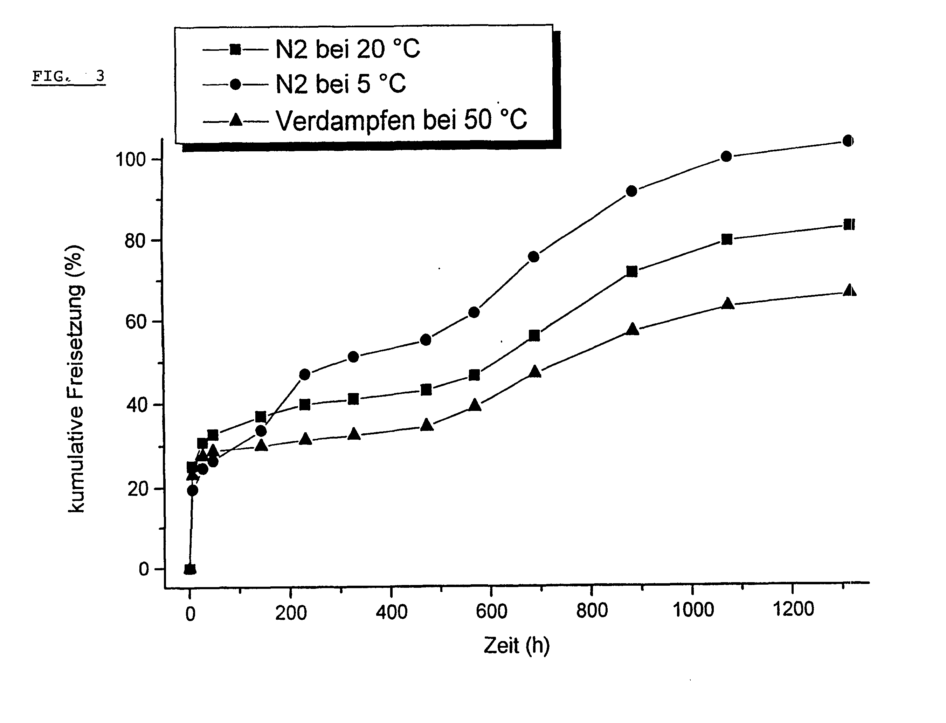

Figur 3 zeigt die Abhängigkeit der in vitro-Freisetzung von Lysozym beim Einblasen von Stickstoff ($N_2$) während der Aushärtung der Mikropartikel im Laborreaktor bei verschiedenen Temperaturen (5°C und 20°C). Weiterhin ist das in vitro-Freisetzungsprofil von Mikropartikeln gezeigt, bei denen das Lösungsmittel während der Aushärtungsphase bei 50°C abgedampft wurde. Dabei ist eine niedrigere Gesamtfreisetzung beim Einsatz von höheren Temperaturen zu erkennen. Weiterhin kommt es durch die Senkung der Temperatur von 20°C auf 5°C zu einer um 6% erniedrigten initialen Freisetzung und einer erhöhten Gesamtfreisetzung von 99,7% gegenüber 79,3% bei 20°C nach 1.074 Stunden Freisetzung. Weiterhin zeigt die Kurve "N$_2$ bei 5°C" eine niedrigere Wirkstofffreisetzung während der "Burst"-Phase.

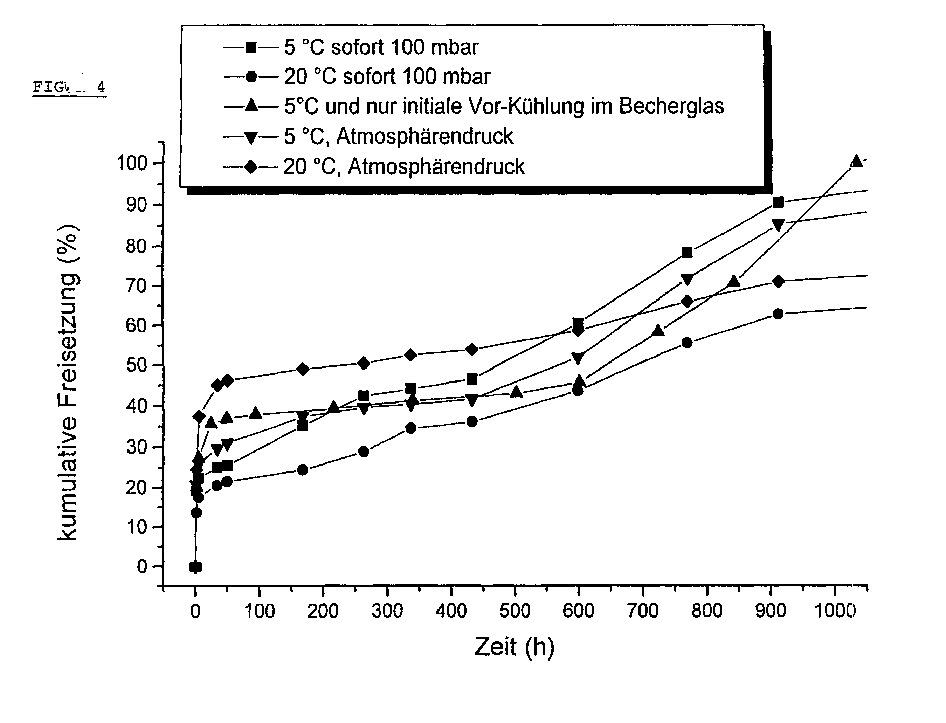

In Figur 4 ist das Ergebnis von Beispiel 9 dargestellt. Durch den Einsatz von niedrigem Druck und niedrigen Temperaturen kommt es zu einem niedrigen "Burst" nach 5 h von 22,4% bei 5°C und 100 mbar Vakuum und zu einer höheren Gesamtfreisetzung von 90,5%. Bei 20°C und 100 mbar Druck beträgt die Gesamtfreisetzung nur 62,8% nach 912 Stunden.

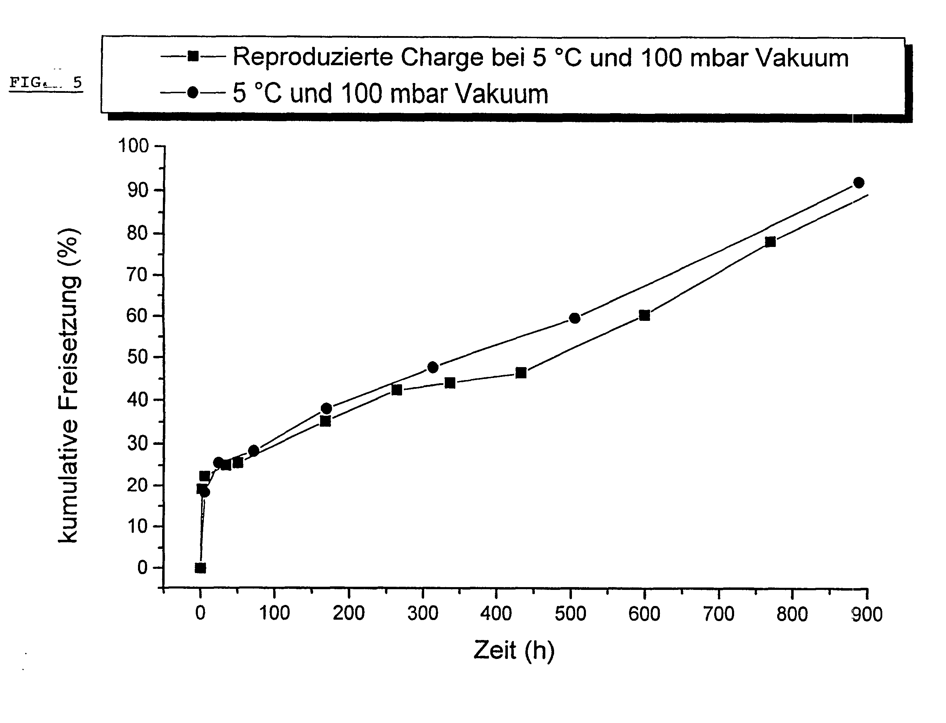

Figur 5 zeigt das Freisetzungsprofil von zwei unabhängig voneinander durchgeführten Ansätzen bei 100 mbar Druck und 5°C während der Aushärtung der Mikropartikel im Laborreaktor. Es können also durch das erfindungsgemäße Verfahren in reproduzierbarer Weise Mikropartikel hergestellt werden, die im wesentlichen das gleiche Freisetzungsprofil aufweisen. Wie aus diesen Datenreihen zu ersehen ist, zeigen die Mikropartikel eine weitgehend lineare Freisetzung.

Die nachfolgenden Beispiele sollen die Erfindung näher erläutern.

**Beispiel 1: Mikropartikelherstellung nach dem W/O/W-Verfahren (Vergleichsbeispiel)**

Mikropartikel mit Lysozym

Zur Herstellung von peptidbeladenen Mikropartikeln aus PLA oder PLGA wurde folgendes "solvent evaporation/extraction"-Verfahren angewendet: Es werden standardmäßig 2,00 g PLGA-Polymer (RG 503 H von der Fa. Boehringer Ingelheim) in einer 20 ml Omnifixspritze mit Luer-Lock und passendem Combiverschlußstopfen in 5,7 ml Dichlormethan (DCM) (Dichte von DCM = 1,32 g/ml [Merck Index]) vollständig gelöst (35% m/v). 100,00 mg/ml Lysozym werden unter leichtem Rühren mittels Magnetrührer in einem 4 ml HPLC-Vial in destilliertem Wasser oder Puffer bis zur Klarheit gelöst. Dann wird 1000 µl der Peptidlösung in die Polymerlösung eingespritzt und mit einem SN-10 G Ultraturrax-Werkzeug 60 sec. lang bei 13.500 Umdrehungen pro Minute (U/min) dispergiert. Die Primäremulsion (W1/O) wird dann aus der Omnifixspritze in 500 ml auf 5°C vorgekühlte, 0,1%ige Polyvinylalkohol(PVA)-Lösung (Mowiol 18-88: Mw = 130 kDa, 88% Hydrolysegrad) eingespritzt und gleichzeitig mit dem SN-18 G Ultraturrax-Werkzeug 60 sec. bei 13.500 U/min dispergiert, so daß eine W1/O/W2-Doppelemulsion entsteht. Diese wird 3 h lang bei Raumtemperatur (RT) im offenen 600 ml Becherglas unter Atmosphärendruck bei 240 U/min mittels IKA-Reihenrührwerk und 2-blättrigen Zentrifugalrührern aushärten gelassen.

Die gesamte Doppelemulsion mit enthaltenen ausgehärteten Mikropartikeln wird dann mit Zentrifugengläsern in der Heraeus Megafuge 1.0 bei 3.000 U/min 3 Minuten lang zentrifugiert und der Überstand an W2-Phase abdekantiert. Anschließend werden die Mikropartikel über eine 500 ml Nutsche (Borosilicat 3.3; Porengröße 4) gegeben und mindestens 3 x mit destilliertem Wasser gewaschen. Dabei werden die Mikropartikel, welche man auf der Fritte erhält, immer wieder mit wenig destilliertem Wasser aufsuspendiert und gewaschen, um PVA-Rückstände zu entfernen.

Die erhaltenen Mikropartikel werden gesammelt und in vorher tarierte Gefäße gegeben und lyophilisiert. Die Mikropartikel werden in die auf Betriebsbedingungen eingeschaltete Delta 1 A Anlage gestellt und einer Haupttrocknung von mindestens 120 h bei -60°C und 0,01 mbar Vakuum unterzogen. Anschließend folgt eine Nachtrocknung über 24 h bei 10°C und 0,01 mbar Vakuum, um letzte Restlösungsmittel- und Wasserreste zu entfernen. Die Mikropartikel werden in den Gefäßen ausgewogen und die Ausbeute berechnet.

**Beispiel 2: Mikropartikelherstellung nach dem S/O/W-Verfahren (Vergleichbeispiel)**

Die Herstellung erfolgt nach den Bedingungen des W/O/W-Verfahrens mit einer Variation im ersten Herstellungsschritt, wobei eine definierte Menge Peptid oder Protein nicht gelöst werden, sondern in lyophilisierter oder sprühgetrockneter Form direkt zu dem gelösten Polymer (35% m/m) in DCM gegeben und mittels SN-10 G Ultraturrax-Werkzeug 30 sec. bei 13.500 U/min dispergiert werden. Diese entstandene S/O- oder Primärsuspension wird dann in der äußeren Phase dispergiert, so daß eine S/O/W-Emulsion entsteht. Die weitere Herstellung folgt analog den Bedingungen des W/O/W-Verfahrens.

**Beispiel 3: Herstellung der Mikropartikel mittels Laborreaktor**

Zur Herstellung von W/O/W- oder S/O/W-Mikropartikeln mittels Prozeßanlage unter kontrollierten Bedingungen wurde ein IKA-Laborreaktor LA-R 1000 verwendet. Es wurden die Bedingungen des W/O/W- oder S/O/W-Verfahrens eingesetzt (siehe Beispiel 1 und 2). Dabei wird die Primäremulsion in einer Omnifixspritze hergestellt und dann durch eine der Öffnungen des Reaktordeckels in die im IKA-Laborreaktor vorgelegte (500 ml), auf eine bestimmte Temperatur voreingestellte, 0,1% PVA-Lösung unter 60 sec. langem Dispergieren mittels Ultraturrax T 25 mit SN 18 G Werkzeug bei 13.500 U/min eingespritzt. Nach Beendigung der Dispergierung wird der Ultraturrax aus dem IKA-Reaktor entfernt und das Reaktorgefäß verschlossen. Nun kann ein bestimmter Druck angelegt werden. In den nachfolgenden Beispielen wurden neben Atmosphärendruck hauptsächlich 500 mbar oder 100 mbar eingesetzt. Anschließend erfolgt die Aushärtung der Mikropartikel unter konstantem Rühren mit einem Ankerrührer bei 40 U/min über 3 h und konstanter Temperatur. Es können verschiedene Temperaturen eingestellt werden. Es wurden meist 20°C oder 5°C eingesetzt. Die Separierung und Lyophilisierung der Mikropartikel erfolgt, wie es für das W/O/W- oder S/O/W-Verfahren bereits beschrieben wurde.

Die Anlage umfaßt ein Reaktorgefäß von 11 Größe und kann über einen doppelwandigen Gefäßboden im Bereich von -30°C bis 180°C temperiert werden. Die Temperierung erfolgt über ein Umwälzthermostat. Das Anlegen von Vakuum erfolgt mit einer MZ 2 C Vakuumpumpe von Jahnke & Kunkel. Weiterhin werden die Temperatur des Reaktorinhalts, der Kühlflüssigkeit, Vakuum, Rührgeschwindigkeit und Umdrehungsgeschwindigkeit des Ultraturrax über Meßfühler (PT 100 für die Temperatur) abgegriffen und an die Software übertragen. Die Steuerung der Prozeßanlage erfolgt über die Software Labworldsoft Version 2.6.

### Beispiel 4: Methode zur Bestimmung der Wirkstoffbeladung der Mikropartikel

Die Bestimmung der Wirkstoffbeladung der Mikropartikel erfolgte nach der modifizierten Methode von Sah et al. (A new strategy to determine the actual Protein Content of Poly(lactide-co-glycolide) Microspheres; Journal of Pharmac. Sciences; 1997; 86; (11); S. 1315-1318). Die Mikropartikel werden in einer Lösung von DMSO/0,5% SDS/0,1 N NaOH gelöst, anschließend wird aus dieser Lösung ein BCA-Assay (Lowry et al. Protein measurement with the Folin Phenol Reagent; J. Biol. Chem.; 193; S. 265-275; 1951) durchgeführt. Damit wird der effektive Beladungsgrad der Mikropartikel bestimmt.

### Beispiel 5: Bestimmung der in vitro-Freisetzung

Die kumulative Freisetzung von Lysozym in % des gesamten in den Mikropartikeln enthaltenen Lysozyms wurde auf folgende Weise untersucht:

Zur Bestimmung der Wirkstofffreisetzung aus den Mikropartikeln wurden je 20 mg Mikropartikel eingewogen (je Charge ein Dreifachansatz). Die Mikropartikel wurden in Pyrexgläser, die einen Schottstopfen GL18-Gewinde mit Teflondichtung aufweisen, gegeben. Die Mikropartikel wurden mit je 5 ml Mc.Ilvaine-Whiting Freisetzungspuffer (Zusammensetzung s. u.) versetzt. Anschließend wurden die Proben in das Freisetzungsgerät gesetzt (6 U/min; 37°C). Das Freisetzungsgerät umfaßt eine Universalhalteplatte aus Polypropylen zur Aufnahme von Eppendorfgefäßen oder Pyrex-Gläsern. Die Platte mit den Gefäßen kann in einem temperierbaren Gehäuse in eine rotierende Bewegung versetzt werden, so daß die Gefäße um ihre Querachse rotieren. Die Drehzahl ist stufenlos von 6-60 U/min einstellbar. Das Temperieren des gesamten Innenraums erfolgt über eine Warmluftzirkulation. Die erste Probe wurde nach circa zwei Stunden entnommen, die zweite nach circa sechs Stunden, die dritte nach circa 24 Stunden, die vierte nach 48 Stunden und die weiteren im Abstand von jeweils drei Tagen. Die Pyrexgläser wurden in der Zentrifuge (Megafuge 1.0, Heraeus, Hanau) bei 3000 U/min (4700 g) 3 Minuten zentrifugiert, anschließend wurde mit einer Pasteurpipette der überstehende Puffer möglichst vollständig abgezogen. Danach wurde wiederum 5 ml Puffer in die Gläser gegeben, und die Proben wurden wieder ins Freisetzungsgerät eingesetzt. Der Puffer wurde vor Licht geschützt und bei 4°C im Kühlschrank aufbewahrt.

Zusammensetzung des Mc.Ilvaine-Whiting Freisetzungspuffers:

0,0094 M Zitronensäure
0,1812 M Dinatriumhydrogenphosphat
0,01% (w/v) Tween 20 für die Molekularbiologie
0,025% (w/v) Natriumazid
pH 7,4
in destilliertem Wasser.

Die abpipettierte Peptidlösung aus den Eppendorf-Gefäßen oder den Pyrexgläsern wurde in 4 ml HPLC-Vials mit durchstechbarer Teflondichtung und Drehverschluß überführt und entweder sofort per HPLC analysiert oder bei -30°C aufbewahrt. Die Proben wurden dann vor der HPLC-Analyse zwei Stunden bei Raumtemperatur aufgetaut und dabei mehrfach per Hand kurz geschüttelt. Es wurde auf eine vollständig klare Lösung nach dem Auftauen geachtet.

Die HPLC-Analyse erfolgte auf einer Waters HPLC mit W600-Pumpe, 717 Autosampler, Satin 474 Fluoreszenzdetektor und Millenium 3.15 Software. Die Einstellungen für Lysozym waren:

- Flußgeschwindigkeit 1 ml/min
- Puffer A = 0,1% TFA (Trifluoressigsäure) in Wasser, Puffer B = 0,1% TFA in Acetonitril
- Gradient: 80% A, 20% B in 10 Minuten auf 60% A, 40% B; bis 12 Minuten auf 80% A, 20% B

- Anregungswellenlänge = 280 nm, Emissionswellenlänge = 340 nm bei Gain = 100, 256 Attention und STD
- Säulenofentemperierung 40°C
- Säule: TSK Gel RP 18, NP; 5 µm; 35 mm x 4,6 mm
- Die Fließmittel wurden vorher per Helium oder Ultraschall entgast und während der Analytik mittels Degaser entgast.
- Pro Sampleset wurden Standardreihen von 0,05 bis 4 µg Lysozym/ml Freisetzungspuffer bei 100 µl Injektionsvolumen und 10 bis 100 µg Lysozym/ml Freisetzungspuffer bei 10 µl Injektionsvolumen als Standard analysiert

Die oben beschriebene Methode zur Bestimmung der in vitro-Freisetzung bezieht sich auf Lysozym als Wirkstoff und ist so nicht für Leuprorelin anwendbar. Zur Bestimmung von anderen Wirkstoffen wie beispielsweise Leuprorelin müssen einige Parameter, wie z.B. die verwendete Säule, die Puffermedien und die verwendete Wellenlänge verändert werden. Diese Änderungen sind aber für den Fachmann selbstverständlich.

**Beispiel 6**

Es wurde der Einfluß von verringertem Druck während der Aushärtung der Mikropartikel im Laborreaktor bei 5°C auf die Verkapselungsausbeute untersucht. Es wurden gemäß Beispiel 3 in einem S/O/W-Verfahren drei Mikropartikelpräparationen unter verschiedenen Bedingungen hergestellt. In Ansatz 1 erfolgte die Aushärtung der Mikropartikel bei Atmosphärendruck, in Ansatz 2 bei 500 mbar, in Ansatz 3 bei 100 mbar. Bei allen Ansätzen erfolgte die Aushärtung bei 5°C. Es wurde die effektive Wirkstoffbeladung der Mikropartikelpräparationen nach dem in Beispiel 4 dargestellten Verfahren ermittelt und daraus die Verkapselungsausbeute (VE) berechnet. Das Ergebnis ist in Figur 1 dargestellt. Die Verkapselungsausbeute steigt mit sinkendem Druck.

**Beispiel 7**

Wie in Beispiel 6 wurden Mikropartikelpräparationen, die unter verschiedenen Bedingungen im Laborreaktor hergestellt worden waren, auf ihre Verkapselungsausbeute hin untersucht. In Ansatz 1 erfolgte die Aushärtung der Mikropartikel unter Atmosphärendruck, bei Ansatz 2 bei 500 mbar. Bei beiden Ansätzen erfolgte die Aushärtung bei 20°C. Anschließend wurde die Verkapselungsausbeute ermittelt. Wie aus Figur 2 entnommen werden kann, steigt auch bei einer Verarbeitungstemperatur von 20°C die Verkapselungsausbeute mit sinkendem Druck.

**Beispiel 8**

Es wurden Mikropartikel nach dem S/O/W-Verfahren unter drei verschiedenen Bedingungen im Laborreaktor hergestellt. In Ansatz 1 und 2 wurde während der Aushärtung der Mikropartikel im Laborreaktor bei 5°C bzw. 20°C Stickstoff eingeblasen. In Ansatz 3 wurde das Lösungsmittel während der Aushärtungsphase bei 50°C abgedampft. Es wurde die in vitro-Freisetzung von Lysozym bei Mikropartikeln der drei Ansätze nach dem in Beispiel 5 dargestellten Verfahren bestimmt.

Das Ergebnis ist in Figur 3 gezeigt. Beim Einsatz von höheren Temperaturen ist eine niedrigere Gesamtfreisetzung zu beobachten. Durch Senkung der Temperatur von 20°C auf 5°C kommt es zu einer um 6% erniedrigten initialen Freisetzung und zu einer erhöhten Gesamtfreisetzung von 99,7% nach 1074 Stunden gegenüber 79,3% bei 20°C.

**Beispiel 9**

Es wurden fünf Mikropartikelpräparationen unter verschiedenen Bedingungen nach dem S/O/W-Verfahren hergestellt:

- 20°C während der Aushärtung der Mikropartikel im Laborreaktor unter Atmosphärendruck ("20°C")
- 5°C während der Aushärtung der Mikropartikel im Laborreaktor unter Atmosphärendruck ("5°C")
- 20°C während der Aushärtung der Mikropartikel im Laborreaktor bei 100 mbar ("20°C sofort 100 mbar")
- 5°C während der Aushärtung der Mikropartikel im Laborreaktor bei 100 mbar ("5°C sofort 100 mbar")
- Nach Beispiel 2 im Becherglas, wobei die äußere Phase auf 5°C vorgekühlt wurde und nach Dispersion der S/O-Phase in der äußeren Phase die S/O/W-Emulsion bei Raumtemperatur und Atmosphärendruck gerührt wurde. Dabei kam es innerhalb von 30 Minuten zu einer Angleichung der Temperatur der aushärtenden Mikropartikel auf Raumtemperatur ("5°C und nur initiale Vor-Kühlung im Becherglas").

Es wurde die in vitro-Freisetzung von Lysozym aus Mikropartikeln der fünf Ansätze bestimmt. Das Ergebnis ist in Figur 4 gezeigt.

Ein Teil der Ergebnisse ist in nachstehender Tabelle 1 zusammengefaßt:

Tabelle 1

| | "Burst" nach 5 h | Gesamtfreisetzung nach 912 h | Linear freigesetzte Menge (Differenz zwischen "Burst" und Gesamtfreisetzung) |
|---|---|---|---|
| S/O/W Becherglas, mit initialer Kühlung von 5°C | 27,5% | 100% | ca. 72,5% |
| Laborreaktor 20°C, 1013 mbar | 37,6% | 71,1% | ca. 33,5% |
| Laborreaktor 5°C, 1013 mbar | 26,1% | 85,5% | ca. 59,5% |
| Laborreaktor 20°C, 100 mbar | 17,6% | 62,8% | ca. 45,2% |
| Laborreaktor 5°C, 100 mbar | 22,4% | 90,5% | ca. 68% |

Beim Ansatz im Becherglas ist ein "Burst" von 27,5% nach 5 Stunden zu sehen. Der "Burst" bei 20°C und 1013 mbar ist mit 37,6% deutlich höher. Werden die aushärtenden Mikropartikel gekühlt, liegt auch der "Burst" niedriger. Weiterhin zeigt sich bei 5°C und 1013 mbar eine deutlich höhere Gesamtfreisetzung von 85,5% als bei 20°C und 1013 mbar nach 912 Stunden Freisetzung. Durch Einsatz von Vakuum kann die Freisetzung in der "Burst"-Phase weiter erniedrigt werden.

**Beispiel 10**

Es wurden zwei Mikropartikelpräparationen unabhängig voneinander im Laborreaktor gemäß dem in Beispiel 3 beschriebenen Verfahren unter identischen Bedingungen hergestellt. Die Bedingungen waren: 5°C und 100 mbar während der Aushärtung der Mikropartikel.

Es wurde die in vitro-Freisetzung der beiden Mikropartikelpräparationen gemäß Beispiel 5 bestimmt. Das Ergebnis ist in Figur 5 gezeigt. Es lassen sich in reproduzierbarer Weise Mikropartikelpräparationen mit im wesentlichen identischen Freisetzungseigenschaften herstellen.

**Beispiel 11**

Einfluß von Druck und Temperatur bei Leuprolin-MP nach dem W/O/W-Verfahren

Es wurde der Einfluß von verringertem Druck und Temperatur während der Aushärtung der Mikropartikel im Laborreaktor bei 5°C auf die Eigenschaften der Mikropartikel untersucht. Es wurden wie in Beispiel 1 beschrieben in einem W/O/W-Verfahren zwei Mikropartikelpräparationen unter verschiedenen Bedingungen hergestellt. Dabei wurde als Wirkstoff Leuprorelinacetat verwendet.

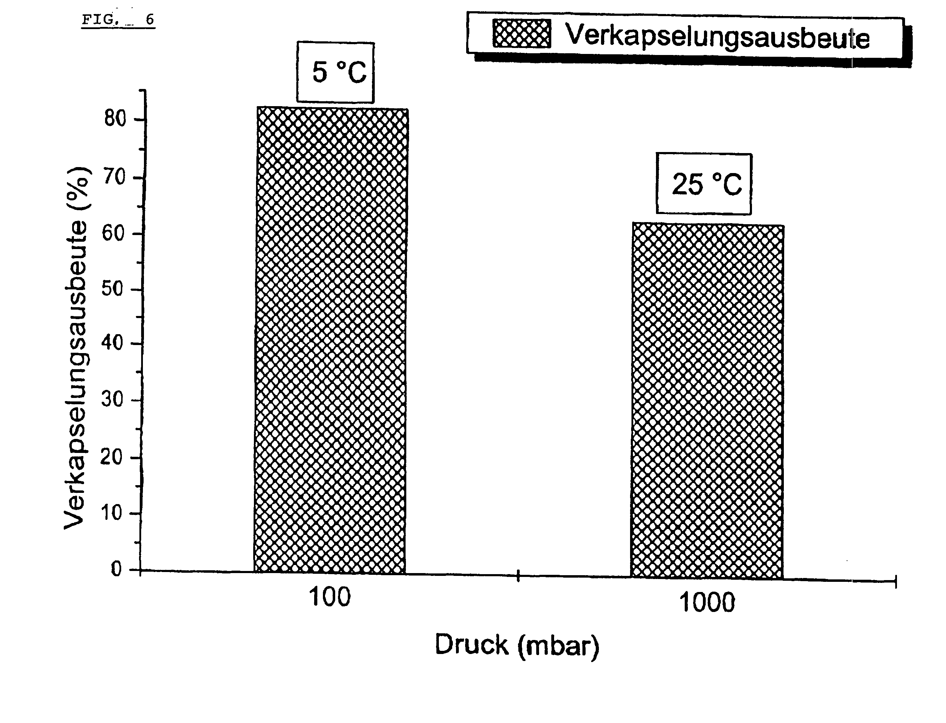

In Ansatz 1 erfolgte die Aushärtung der Mikropartikel bei 5°C und 100 mbar und in Ansatz 2 bei 25°C und 1000 mbar. Es wurde die effektive Wirkstoffbeladung der Mikropartikelpräparationen wie in dem in Beispiel 4 näher dargestellten Verfahren ermittelt und daraus die Verkapselungsausbeute (VE) berechnet. Das Ergebnis ist in Figur 6 dargestellt. Die Verkapselungsausbeute steigt mit sinkendem Druck.

**Beispiel 12**

Einfluß von Druck, Temperatur und Zugabe von Chitosan

Es wurde der Einfluß von verringertem Druck und Temperatur während der Aushärtung der Mikropartikel im Laborreaktor bei 5°C auf die Eigenschaften der Mikropartikel untersucht. Es wurden wie in Beispiel 1 beschrieben in einem W/O/W-Verfahren eine Mikropartikelpräparation mit dem Zusatz an Chitosan (MW = 150.000) hergestellt. Dabei wurde als Wirkstoff Leuprorelinacetat verwendet.

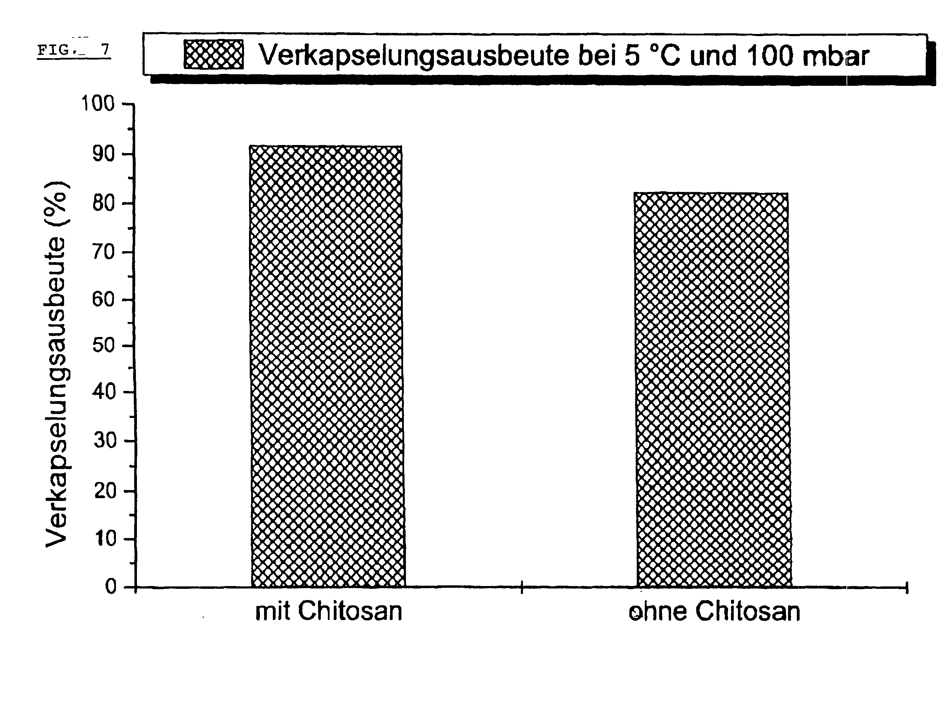

In Ansatz 1 erfolgte die Aushärtung der Mikropartikel bei 5°C und 100 mbar. Es wurde die effektive Wirkstoffbeladung der Mikropartikelpräparationen nach dem in Beispiel 4 dargestellten Verfahren ermittelt und daraus die Verkapselungsausbeute (VE) berechnet. Das Ergebnis ist in Figur 7 dargestellt.

Hierbei ist zu erkennen, daß gegenüber Ansatz 1, Beispiel 11 (Herstellung nach W/O/W ohne Zusatz von Chitosan, aber mit Vakuum- und Temperatureinsatz) eine erhöhte VE und eine verzögerte Freisetzung erfolgt. Dieser Ansatz belegt, daß durch die Zugabe von Chitosan noch bessere Ergebnisse erzielt werden können.

**Beispiel 13**

Einfluß von Druck und Temperatur bei Leuprorelinacetat-Mikropartikeln nach dem S/O/W-Verfahren

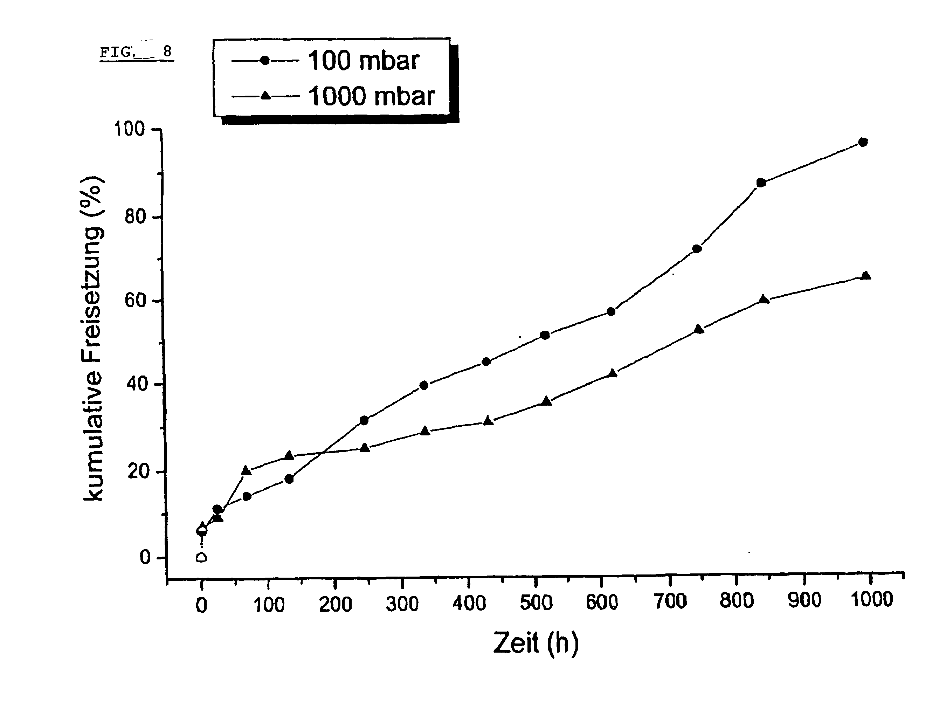

Es wurde der Einfluß von verringertem Druck und Temperatur während der Aushärtung der Mikropartikel im Laborreaktor bei 5°C auf die Eigenschaften der Mikropartikel untersucht. Es wurden gemäß Beispiel 2 in einem S/O/W-Verfahren zwei Mikropartikelpräparationen unter verschiedenen Bedingungen hergestellt. Dabei wurde als Wirkstoff Leuprorelinacetat verwendet. In Ansatz 1 erfolgte die Aushärtung der Mikropartikel bei 5°C und 100 mbar und in Ansatz 2 bei 25°C und 1000 mbar. Es wurde die effektive Wirkstoffbeladung der Mikropartikelpräparationen nach dem in Beispiel 4 dargestellten Verfahren ermittelt und daraus die Verkapselungsausbeute (VE) berechnet. Die VE ist bei Anwendung von Vakuum und erniedrigter Temperatur um den Faktor 2,25 höher. In Figur 8 ist die in-vitro Freisetzung dieser Mikropartikel mit Leuprorelinacetat dargestellt.

**Patentansprüche**

1. Verfahren zur Herstellung von Mikropartikeln zur verzögerten Freisetzung eines Wirkstoffs, **dadurch gekennzeichnet, daß**

   a) eine den Wirkstoff enthaltende Zusammensetzung zu einer organischen Lösung eines Polymers gegeben wird und darin dispergiert wird,

   b) die in a) entstehende Emulsion oder Dispersion in eine äußere Phase gegeben wird und darin dispergiert wird, wobei die äußere Phase zum Zeitpunkt der Zugabe eine Temperatur von 0°C bis 20°C hat, und

   c) das organische Lösungsmittel entfernt wird, indem die in b) entstehende Dispersion oder Emulsion einem Druck von weniger als 1.000 mbar ausgesetzt wird, oder indem ein inertes Gas in die in b) entstehende Dispersion oder Emulsion eingeleitet wird.

2. Verfahren nach Anspruch 1, **dadurch gekennzeichnet, daß** die Temperatur 0°C bis 10°C ist.

3. Verfahren nach Anspruch 2, **dadurch gekennzeichnet, daß** die Temperatur 3°C bis 7°C ist.

4. Verfahren nach einem der Ansprüche 1 bis 3, **dadurch gekennzeichnet, daß** die in Schritt b) entstehende Dispersion oder Emulsion während der Entfernung des organischen Lösungsmittels weiterhin auf eine Temperatur von 0°C bis 20°C temperiert wird.

5. Verfahren nach Anspruch 4, **dadurch kennzeichnet, daß** die in Schritt b) entstehende Dispersion oder Emulsion während der Entfernung des organischen Lösungsmittels weiterhin auf eine Temperatur von 0°C bis 10°C temperiert wird.

6. Verfahren nach einem der Ansprüche 1 bis 5, **dadurch gekennzeichnet, daß** das organische Lösungsmittel entfernt wird, indem die in Schritt b) entstehende Dispersion oder Emulsion einem Druck von 50 bis 150 mbar ausgesetzt wird.

7. Verfahren nach einem der Ansprüche 1 bis 5, **dadurch gekennzeichnet, daß** das organische Lösungsmittel entfernt wird, indem ein inertes Gas, vorzugsweise Stickstoff in die in b) entstehende Dispersion oder Emulsion eingeleitet wird.

8. Verfahren nach einem der Ansprüche 1 bis 7, **dadurch gekennzeichnet, daß** als Polymer Polymilchsäure, Polyglykolsäure oder ein Milchsäure-Glykolsäure-Copolymer verwendet wird.

9. Verfahren nach einem der Ansprüche 1 bis 8, **dadurch gekennzeichnet, daß** die organische Lösung eines Polymers als Lösungsmittel Dichlormethan enthält.

10. Verfahren nach einem der Ansprüche 1 bis 9, **dadurch gekennzeichnet, daß** die Polymerkonzentration in der organischen Lösung eines Polymers 5 bis 50% (w/v) ist.

11. Verfahren nach einem der Ansprüche 1 bis 10, **dadurch gekennzeichnet, daß** die den Wirkstoff enthaltende Zusammensetzung eine wäßrige Lösung ist.

12. Verfahren nach einem der Ansprüche 1 bis 10, **dadurch gekennzeichnet, daß** die den Wirkstoff enthaltende Zusammensetzung aus Feststoffen besteht.

13. Verfahren nach Anspruch 12, wobei die den Wirkstoff enthaltende Zusammensetzung **dadurch** bereitgestellt wird, daß eine den Wirkstoff enthaltende Lösung sprühgetrocknet wird.

14. Verfahren nach einem der Ansprüche 1 bis 13, **dadurch gekennzeichnet, daß** als äußere Phase eine wäßrige Lösung eingesetzt wird.

15. Verfahren nach Anspruch 14, **dadurch gekennzeichnet, daß** die wäßrige äußere Phase einen Emulgator und/ oder ein Schutzkolloid enthält.

16. Verfahren nach Anspruch 15, **dadurch gekennzeichnet, daß** das Schutzkolloid ausgewählt ist aus der Gruppe bestehend aus Polyvinylalkohol, Polyvinylpyrrolidon und Polyethylenglykol.

17. Verfahren nach einem der Ansprüche 1 bis 13, **dadurch gekennzeichnet, daß** die äußere Phase eine nicht-wäßrige Phase ist, die einen Emulgator und/oder ein Schutzkolloid enthält.

18. Verfahren nach Anspruch 17, **dadurch gekennzeichnet, daß** die äußere Phase Span, Tween oder Brij enthält.

19. Verfahren nach einem der Ansprüche 1 bis 18, **dadurch gekennzeichnet, daß** die den Wirkstoff enthaltende Zusammensetzung weiterhin Chitosan enthält.

20. Mikropartikel, erhältlich durch ein Verfahren nach einem der Ansprüche 1 bis 19.

21. Mikropartikel nach Anspruch 20, **dadurch gekennzeichnet, daß** gemäß dem in vitro-Freisetzungsprofil der Mikropartikel

  a) innerhalb von 24 Stunden ab dem Beginn der Freisetzung weniger als 25% der gesamten Wirkstoffmenge freigesetzt werden;

  b) innerhalb von 900 Stunden ab dem Beginn der Freisetzung wenigstens 80% der gesamten Wirkstoffmenge freigesetzt werden; und

  c) die Freisetzung in dem Zeitraum zwischen 24 Stunden und 900 Stunden nach Beginn der Freisetzung im wesentlichen nach einer Kinetik nullter Ordnung erfolgt.

22. Mikropartikel nach Anspruch 21, **dadurch gekennzeichnet, daß** gemäß dem in vitro-Freisetzungsprofil der Mikropartikel innerhalb von 24 Stunden ab dem Beginn der Freisetzung weniger als 20% der gesamten Wirkstoffmenge freigesetzt werden.

23. Mikropartikel nach Anspruch 21 oder 22, **dadurch gekennzeichnet, daß** gemäß dem in vitro-Freisetzungsprofil der Mikropartikel innerhalb von 900 Stunden ab dem Beginn der Freisetzung wenigstens 90% der gesamten Wirkstoffmenge freigesetzt werden.

24. Mikropartikel nach einem der Ansprüche 21 bis 23, **dadurch gekennzeichnet, daß** im Zeitraum zwischen 48 Stunden und 900 Stunden nach Beginn der Freisetzung täglich 1,75% bis 2,5% der gesamten Wirkstoffmenge freigesetzt werden.

25. Mikropartikel nach einem der Ansprüche 20 bis 24, **dadurch gekennzeichnet, daß** als physiologisch aktiver Wirkstoff ein Peptid oder Protein enthalten ist.

26. Arzneimittel enthaltend Mikropartikel nach einem der Ansprüche 20 bis 25.

27. Arzneimittel nach Anspruch 26, **dadurch gekennzeichnet, daß** es für die parenterale Verabreichung hergerichtet ist.

**Claims**

1. Method for manufacturing microparticles for delayed release of an active ingredient, **characterized in that**

   a) a composition containing the active ingredient is added to an organic solution of a polymer and dispersed therein,

   b) the emulsion or dispersion produced in a) is added to an outer phase and dispersed therein, wherein said the temperature of the outer phase at the time of addition is between 0°C and 20°C, and

   c) the organic solvent is removed by subjecting the dispersion or emulsion produced in b) to a pressure of less than 1,000 mbar, or by conducting an inert gas into the dispersion or emulsion produced in b).

2. Method according to claim 1, **characterized in that** the temperature is between 0°C and 10°C.

3. Method according to claim 2, **characterized in that** the temperature is between 3°C and 7°C.

4. Method according to one of the claims 1 to 3, **characterized in that** the dispersion or emulsion produced in step b) continues to be regulated at a temperature of between 0°C and 20°C during removal of the organic solvent.

5. Method according to claim 4, **characterized in that** the dispersion or emulsion produced in step b) continues to be regulated at a temperature of between 0°C and 10°C during removal of the organic solvent.

6. Method according to one of claims 1 to 5, **characterized in that** the organic solvent is removed by subjecting the dispersion or emulsion produced instep b) to a pressure of 50 to 150 mbar.

7. Method according to one of claims 1 to 5, **characterized in that** the organic solvent is removed by conducting an inert gas, preferably nitrogen, into the dispersion or emulsion produced in b).

8. Method according to one of claims 1 to 7, **characterized in that** a polymer in the form of polylactic acid, polyglycolic acid or a lactic acid-glycolic acid-copolymer is used.

9. Method according to one of claims 1 to 8, **characterized in that** the organic solution of a polymer contains a solvent in the form of dichloromethane.

10. Method according to one of claims 1 to 9, **characterized in that** the polymer concentration in the organic solution of a polymer is 5 to 50 % (w/v).

11. Method according to one of claims 1 to 10, **characterized in that** the composition containing the active ingredient is an aqueous solution.

12. Method according to one of claims 1 to 10, **characterized in that** the composition containing the active ingredient consists of solids.

13. Method according to claim 12, in which the composition containing the active ingredient is prepared by spray-drying a solution containing the active ingredient.

14. Method according to one of claims 1 to 13, **characterized in that** an aqueous solution is used as the outer phase.

15. Method according to claim 14, **characterized in that** the aqueous outer phase contains an emulsifier and/or a protective colloid.

16. Method according to claim 15, **characterized in that** the protective colloid is selected from the group consisting of polyvinyl alcohol, polyvinylpyrrolidone and polyethylene glycol.

17. Method according to one of claims 1 to 13, **characterized in that** said outer phase is a non-aqueous phase containing an emulsifier and/or a protective colloid.

**18.** Method according to claim 17, **characterized in that** said outer phase contains Span, Tween or Brij.

**19.** Method according to one of claims 1 to 18, **characterized in that** the composition containing the active ingredient also contains chitosan.

**20.** Microparticles obtained by a method according to one of claims 1 to 19.

**21.** Microparticles according to claim 20, **characterized in that** in accordance with the in vitro-release profile of said microparticles

a) less than 25 % of the total amount of active ingredient is released within 24 hours of the onset of release;

b) at least 80 % of the total amount of active ingredient is released within 900 hours of the onset of release, and

c) the release during the period between 24 hours and 900 hours of the onset of release is kinetically substantially on the order of zero.

**22.** Microparticles according to claim 21, **characterized in that** in accordance with the in vitro-release profile of said microparticles, less than 20 % of the total amount of active ingredient is released within 24 hours of the onset of release.

**23.** Microparticles according to claim 21 or 22, **characterized in that** in accordance with the in vitro-release profile of said microparticles, at least 90 % of the total amount of active ingredient is released within 900 hours of the onset of release.

**24.** Microparticles according to one of claims 21 to 23, **characterized in that** during the period between 48 and 900 hours of onset of release, 1.75 % to 2.5 % of the total amount of active ingredient is released daily.

**25.** Microparticles according to one of the claims 20 to 24, **characterized in that** contained therein is a physiologically active substance in the form of a peptide or protein.

**26.** Pharmaceutical containing microparticles according to one of claims 20 to 25.

**27.** Pharmaceutical according to claim 26, **characterized in that** it is prepared for parenteral administration.


**Revendications**

**1.** Procédé de fabrication de microparticules pour la libération prolongée d'un principe actif, **caractérisé en ce que**

a) une composition contenant le principe actif est ajoutée à une solution organique d'un polymère et est dispersée dans celle-ci,
b) l'émulsion ou la dispersion obtenue au point a) est ajoutée dans une phase externe et est dispersée dans celle-ci, la phase externe présentant au moment de l'addition, une température de 0° C à 20° C, et
c) le solvant organique est éliminé en soumettant la dispersion ou l'émulsion obtenue au point b) à une pression inférieure à 1 000 bar ou en introduisant un gaz inerte dans la dispersion ou l'émulsion obtenue au point b).

**2.** Procédé selon la revendication 1, **caractérisé en ce que** la température est de 0° C à 10° C.

**3.** Procédé selon la revendication 2, **caractérisé en ce que** la température est de 3° C à 7°C.

**4.** Procédé selon l'une quelconque des revendications 1 à 3, **caractérisé en ce que** la dispersion ou l'émulsion obtenue à l'étape b) est en outre tempérée pendant l'élimination du solvant organique à une température de 0° C à 20° C.

**5.** Procédé selon la revendication 4, **caractérisé en ce que** la dispersion ou l'émulsion obtenue à l'étape b) est en outre tempérée pendant l'élimination du solvant organique à une température de 0° C à 10° C.

**6.** Procédé selon l'une quelconque des revendications 1 à 5, **caractérisé en ce que** le solvant organique est éliminé, en soumettant la dispersion ou l'émulsion obtenue à l'étape b) à une pression de 50 à 150 mbar.

**7.** Procédé selon l'une quelconque des revendications 1 à 5, **caractérisé en ce que** le solvant organique est éliminé en introduisant un gaz inerte, de préférence de l'azote, dans la dispersion ou l'émulsion obtenue à l'étape b).

**8.** Procédé selon l'une quelconque des revendications 1 à 7, **caractérisé en ce que** l'on utilise comme polymère, de l'acide polylactique, de l'acide polyglycolique ou un copolymère d'acide lactique-acide glycolique.

**9.** Procédé selon l'une quelconque des revendications 1 à 8, **caractérisé en ce que** la solution organique d'un polymère contient comme solvant, du dichlorométhane.

**10.** Procédé selon l'une quelconque des revendications 1 à 9, **caractérisé en ce que** la concentration en polymère dans la solution organique d'un polymère est de 5 à 50 % (p/v).

**11.** Procédé selon l'une quelconque des revendications 1 à 10, **caractérisé en ce que** la composition contenant le principe actif est une solution aqueuse.

**12.** Procédé selon l'une quelconque des revendications 1 à 10, **caractérisé en ce que** la composition contenant le principe actif est constituée de substances solides.

**13.** Procédé selon la revendication 12, dans lequel la composition contenant le principe actif est préparée en séchant par pulvérisation une solution contenant le principe actif.

**14.** Procédé selon l'une quelconque des revendications 1 à 13, **caractérisé en ce que** l'on utilise comme phase externe, une solution aqueuse.

**15.** Procédé selon la revendication 14, **caractérisé en ce que** la phase aqueuse externe contient un émulsifiant et/ ou un colloïde protecteur.

**16.** Procédé selon la revendication 15, **caractérisé en ce que** le colloïde protecteur est choisi dans le groupe constitué d'alcool de polyvinyle, de polyvinylpyrrolidone et de polyéthylèngeglycol.

**17.** Procédé selon l'une quelconque des revendications 1 à 13, **caractérisé en ce que** la phase externe est une phase non aqueuse qui contient un émulsifiant et/ou un colloïde protecteur.

**18.** Procédé selon la revendication 17, **caractérisé en ce que** la phase externe contient du Span, du Tween ou du Brij.

**19.** Procédé selon l'une quelconque des revendications 1 à 18, **caractérisé en ce que** la composition contenant le principe actif contient en outre du chitosan.

**20.** Microparticules disponibles par un procédé selon l'une quelconque des revendications 1 à 19.

**21.** Microparticules selon la revendication 20, **caractérisées en ce que**, selon le profil de libération *in vitro* des microparticules

    a) en l'espace de 24 heures à partir du début de la libération, moins de 25 % de la quantité totale de principe actif ont été libérés ;
    b) en l'espace de 900 heures à partir du début de la libération, moins de 80 % de la quantité totale de principe actif ont été libérés ; et
    c) la libération, dans la durée entre 24 heures et 900 heures après le début de la libération suit essentiellement une cinétique d'ordre zéro.

**22.** Microparticules selon la revendication 21, **caractérisées en ce que** selon le profil de libération *in vitro* des microparticules, en l'espace de 24 heures après le début de la libération, moins de 20 % de la quantité totale de principe actif sont libérés.

**23.** Microparticules selon la revendication 21 ou 22, **caractérisé en ce que** selon le profil de libération *in vitro* des microparticules, en l'espace de 900 heures à partir du début de la libération, moins de 90 % de la quantité totale de principe actif sont libérés.

24. Microparticules selon l'une quelconque des revendications 21 à 23, **caractérisées en ce que** dans la durée entre 48 heures et 900 heures après le début de la libération, quotidiennement, 1,75 % à 2,5 % de la quantité totale de principe actif sont libérés.

25. Microparticules selon l'une quelconque des revendications 20 à 24, **caractérisées en ce qu'**elles contiennent comme principe physiologiquement actif, un peptide ou une protéine.

26. Médicament contenant des microparticules selon l'une quelconque des revendications 20 à 25.

27. Médicament selon la revendication 26, **caractérisée en ce qu'**il est destiné à l'administration parentérale.

FIG. 1

Verkapselungsausbeute bei 5 °C

Druck (mbar)

EP 1 341 522 B1

FIG. 2

**Verkapselungsausbeute bei 20 °C**

Verkapselungsausbeute (%)

Druck (mbar)

EP 1 341 522 B1

FIG. 3

Kumulative Freisetzung (%)

Zeit (h)

N2 bei 20 °C
N2 bei 5 °C
Verdampfen bei 50 °C

FIG. 4

*Legend:*
- 5 °C sofort 100 mbar
- 20 °C sofort 100 mbar
- 5°C und nur initiale Vor-Kühlung im Becherglas
- 5 °C, Atmosphärendruck
- 20 °C, Atmosphärendruck

*Y-axis:* kumulative Freisetzung (%)

*X-axis:* Zeit (h)

EP 1 341 522 B1

FIG. 5

FIG. 6

FIG. 7 — Verkapselungsausbeute bei 5 °C und 100 mbar

EP 1 341 522 B1

FIG. 8